# FASCICULE DE BREVET EUROPEEN

(11) **EP 0 608 519 B1**
(45) Date de publication et mention de la délivrance du brevet: **04.11.1998**
(21) Numéro de dépôt: 93119624.0
(22) Date de dépôt: 06.12.1993
(51) Int. Cl.: B04B 7/08, B04B 5/04

(54) **Séparateur centrifuge**
Zentrifugalseparator
Centrifugal separator

(30) Priorité: 29.01.1993 CH 276/93
(43) Date de publication de la demande: 03.08.1994
(73) Titulaire: MEDTRONIC ELECTROMEDICS INC, Parker, Colorado 80134 (US)
(72) Inventeur: Rochat, Jean-Denis, CH-1295 Mies (CH)
(74) Mandataire: Micheli & Cie

(56) Documents cités:
- US-A- 4 204 537
- US-A- 4 300 717
- US-A- 4 889 524

## Description

La présente invention se rapporte à un séparateur centrifuge pour la séparation des constituants du sang (aphérèse) ou d'autres fluides biologiques, et qui est destiné a être incorporé par exemple dans un appareil pour la récupération et la filtration stérile du sang pré- et post-opératoire en vue d'une éventuelle auto-transfusion.

Dans le type d'appareil précité, le sang récupéré est aspiré du champ opératoire et mélangé à une solution anti-coagulante, puis provisoirement conservé dans un réservoir filtrant. Ce sang est alors pompé vers le bol d'une centrifugeuse où la séparation et l'accumulation des cellules rouges se produisent à une vitesse de centrifugation élevée (env. 6000 t/min). Quand le bol est plein, les globules rouges sont lavés à l'aide d'une solution stérile, les autres constituants étant recueillis dans une poche de récupération. A la fin de la phase de lavage, les globules rouges sont transférés dans une poche de réinfusion, en attente d'une utilisation ultérieure pour le patient.

On connaît déjà des séparateurs centrifuges pour de tels appareils, par exemple ceux décrits dans les brevets US 4.300.717, 4.795.419 et 4.889.524, qui comportent un corps central monté dans un bol rotatif. Ces séparateurs présentent toutefois des inconvénients, notamment en ce qui concerne leur mode de fixation dans le séparateur et d'entraînement en rotation, qui sont relativement complexes et coûteux. Un but de la présente invention est donc de fournir un séparateur centrifuge qui soit de conception plus simple et moins coûteuse que ceux connus, tout en permettant d'obtenir des performances similaires à ceux-ci. Un autre but consiste également à améliorer les conditions de récupération des constituants après séparation.

Le séparateur centrifuge selon l'invention vise à atteindre les buts précités et présente les caractéristiques définies dans la revendication 1.

Le dessin annexé illustre schématiquement et à titre d'exemple une forme d'exécution du séparateur centrifuge selon l'invention.

La figure 1 en est une vue générale en coupe verticale.

La figure 2 est une vue en coupe transversale d'un détail agrandi de la partie supérieure du séparateur.

Les figures 3 et 4 sont des vues en coupe horizontale du bol rotatif selon les lignes respectivement III-III et IV-IV de la figure 1.

La figure 5 est une vue partielle en coupe verticale d'une autre réalisation du séparateur selon la figure 1, dans laquelle le bol et sa coupelle d'entraînement sont cylindriques.

La figure 6 est une vue partielle en coupe verticale d'une variante de la figure 5.

Les figures 7 et 8 sont des vues partielles en perspective respectivement de dessus d'une variante de la partie inférieure du bol de centrifugation.

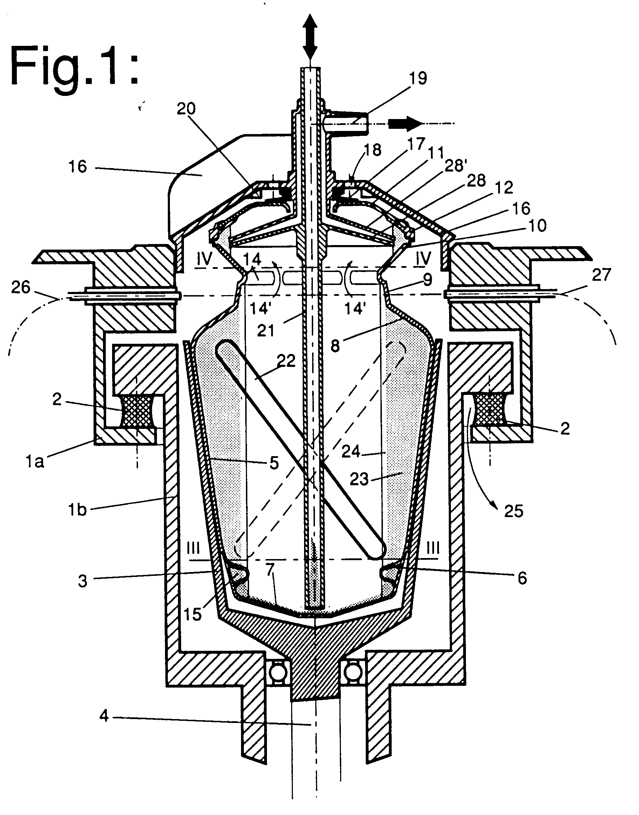

Le séparateur centrifuge selon l'invention, tel qu'il est illustré à titre d'exemple sur la figure 1 comporte une enceinte formée de deux parties 1a, 1b reliées l'une à l'autre par des éléments élastiques 2 faisant office de suspension antivibratoire entre ces deux parties. A l'intérieur de cette enceinte est montée une coupelle conique d'entraînement en rotation 3, prolongée vers le bas par un axe de rotation 4, lui-même entraîné en rotation par un moteur (non montré). Le récipient 1a, 1b et la coupelle d'entraînement 3, de préférence réalisés en métal, par exemple en aluminium, constituent la partie permanente, non jetable, du séparateur centrifuge, qui est destinée à être incorporée par exemple dans un appareil de traitement du sang.

L'élément actif du séparateur centrifuge selon l'invention est constitué par un bol de centrifugation 5 en un matériau plastique, de préférence réalisé par extrusion-soufflage d'une résine synthétique. Ce bol 5 présente une forme générale conique, avec un diamètre croissant vers le haut; il présente dans sa partie inférieure un rétrécissement formant à l'intérieur du bol un renflement annulaire 6 à proximité du fond 7, lui-même légèrement conique. Quant à la partie supérieure du bol 5, elle présente d'abord une zone 8 ayant une conicité inverse prolongée vers le haut par un col également légèrement conique 9, et enfin une portion d'extrémité ouverte 10 s'évasant vers le haut.

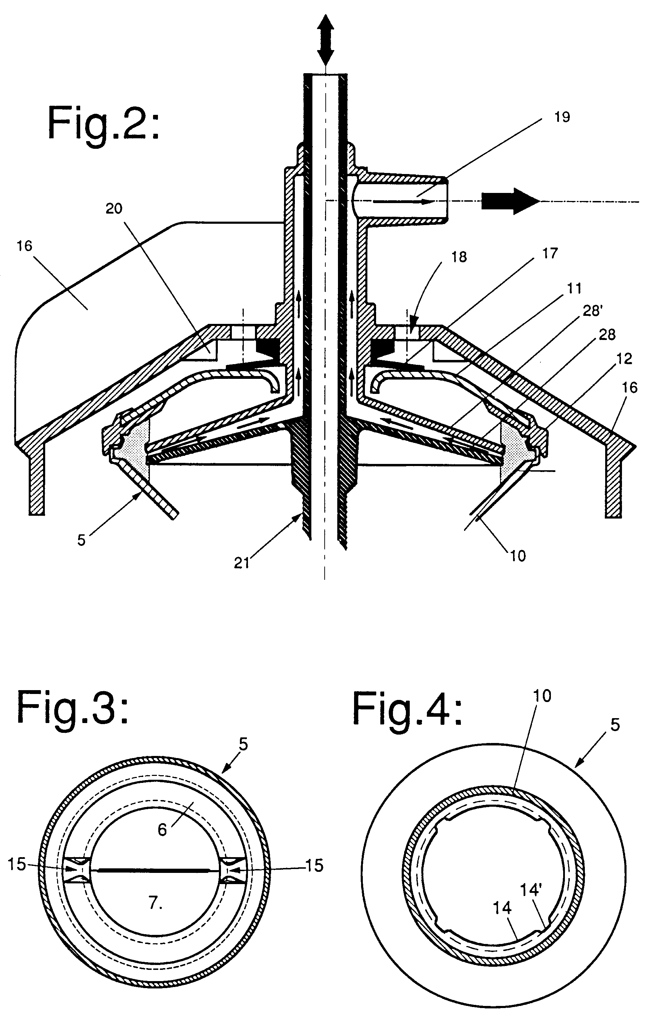

Dans la position de service illustrée sur la figure 1, le bol de centrifugation 5 est muni à son extrémité supérieure 10 d'un couvercle 11, par exemple en acier inoxydable, une pièce intermédiaire 12 assurant la fixation de ce couvercle 11 sur ledit bol 5 et l'étanchéité entre ces deux éléments (voir figure 2). La fixation de pièce intermédiaire 12 sur ladite extrémité 10 du bol 5 est obtenue par exemple au moyen d'une soudure par ultrasons.

Le bol de centrifugation 5 présente encore de fabrication un bourrelet annulaire supérieur 14, dans lequel sont pratiqués des passages verticaux 14' servant de "déversoirs" (par exemple au nombre de 4) répartis de manière régulière sur la circonférence dudit bourrelet (voir figure 4). De même, le renflement interne inférieur 6 présente deux passages verticaux 15 diamétralement opposés, comme montré sur la figure 3.

Le séparateur centrifuge selon l'invention est fermé à son extrémité supérieure par un couvercle 16 fixé de manière amovible par un système classique du type à baïonnette sur la partie supérieure 1a du récipient, et qui comporte sur sa face interne un joint rotatif souple 17, par exemple en élastomère, coopérant en position de service avec la surface supérieure du couvercle de bol 11 pour assurer l'étanchéité lors de la rotation de celui-ci à l'intérieur du récipient fixe. Ce couvercle 16 du séparateur présente encore des ouvertures 18 pour permettre une ventilation de la zone de contact entre le joint rotatif 17 et le couvercle de bol 11 ainsi que de celui-ci. D'autre part, il forme, de même que le bol 5, une partie jetable du dispositif.

Cette partie jetable comprend également un tube d'introduction 21 disposé verticalement à travers le couvercle 16 et solidairement de celui-ci, et dont l'extrémité inférieure ouverte est située en position de service à proximité du fond 6 du bol centrifugateur 5, ainsi que deux éléments circulaires coniques 28,28', formant des lèvres d'aspiration, et un conduit de sortie 19 communiquant avec l'espace séparant lesdites lèvres d'aspiration. Enfin, des ailettes de mélange 22 sont prévues à l'intérieur du bol 5, qui sont de préférence venues de fabrication par soufflage avec la paroi du bol 5.

Le chargement du bol 5 dans le séparateur et le verrouillage du couvercle 16 peuvent être effectués d'une seule main. Le mécanisme de fixation à baïonnette permet de descendre l'ensemble couvercle 16 - bol 5 - coupelle 3 en forçant élastiquement par une butée 20 sur les suspensions 2, puis de remonter et de verrouiller cet ensemble dans la position illustrée sur la figure 1, la butée 20 n'étant alors plus en contact avec le couvercle 11 du bol 5, ce dernier restant par contre en contact avec le joint élastique rotatif 17.

Un des éléments essentiels de la présente invention est constitué par la géométrie particulière du bol de centrifugation 5. Tout d'abord, la forme générale conique du bol (environ 2 à 10 degrés) permet un centrage parfait de celui-ci dans la coupelle 3 et son entraînement efficace par celle-ci. Le bol 5 est forcé dans la coupelle conique 3 avec une force de l'ordre de 20 Newton. Le fond 7 du bol 5 n'est pas en contact avec le fond de la coupelle 3, de telle sorte que sa tolérance de hauteur n'entre pas en compte dans la plage de fonctionnement du joint rotatif souple 17.

Dans les séparateurs centrifuges connus, le bol rotatif est associé à un noyau destiné notamment à permettre d'augmenter la vitesse d'introduction et de stabiliser les remous créés par cette introduction. Grâce à la géométrie particulière du séparateur selon l'invention, et surtout du bol centrifugateur de celui-ci, il a été possible de se passer complètement d'un tel noyau. En effet, le bourrelet annulaire 6 à proximité du fond 7 du bol 5 sert de barrage de stabilisation à l'introduction du fluide à traiter par l'extrémité inférieure du tube 21, ceci afin de limiter les perturbations sur tout la surface de séparation. Par contre, les deux passages verticaux 15 pratiqués dans ce bourrelet inférieur 6 sont destinés à créer une zone turbulente utile dans la phase de lavage. D'autre part, toujours en ce qui concerne la géométrie du bol 5, les passages verticaux 14' répartis de manière régulière dans le bourrelet supérieur 14 constituent un déversoir qui permet d'uniformiser la sortie du fluide sur tout le pourtour du bol et de s'affranchir de l'excentricité. Enfin, les deux disques 28,28' solidaires du tube d'introduction 21 et du couvercle de séparateur 16 présentent de préférence des angles légèrement différents de manière à réduire l'espace entre les deux lèvres vers leur périphérie, ce qui permet de réduire la vitesse tangentielle élevée du fluide à aspirer (due à la force centrifuge) et d'assurer ainsi une évacuation sans remous vers le centre puis vers le conduit de sortie 19.

En ce qui concerne le fonctionnement du séparateur centrifuge selon l'invention, on distingue les deux phases suivantes:
a) Le remplissage : le mélange à traiter (par exemple du sang) est introduit par le tube 21 dans le fond 7 du bol centrifugateur 5, de telle sorte que ce fluide bute d'abord contre le bourrelet inférieur 6 et ne passe que par les deux canaux d'introduction 15, ceci sans perturber la couche de séparation, c'est-à-dire en régime aussi laminaire que possible. Le bol 5 étant entraîné en rotation à une vitesse de l'ordre de 10.000 t/min, les globules rouges 23 sont piégés à proximité des parois dudit bol et le liquide surnageant 24 (plasma) déborde en haut du bol 5 par les déversoirs de régularisation 14', et sort du dispositif par le conduit de sortie 19 (via les lèvres d'aspiration 28,28') pour être récupéré.
   Le contrôle du niveau de remplissage du bol 5 est obtenu par exemple au moyen d'un faisceau optique formé au niveau de la portion supérieure 9 (juste au-dessous du bourrelet supérieur 14) dudit bol, entre un émetteur 26 et un récepteur 27 disposés dans la partie supérieure 1a du séparateur de telle sorte que ledit faisceau soit légèrement décentré pour éviter le tube d'introduction 21. Le passage des premiers globules rouges produit une coupure du faisceau optique et détermine ainsi le moment où le bol 5 est plein de ceux-ci. On réduit alors la vitesse de rotation du bol de moitié environ jusqu'à une seconde détection de globules rouges par coupure de faisceau optique, qui déclenche alors l'arrêt du remplissage, le bol 5 étant plein à environ 80% d'hématocrites. L'épaisseur de plasma étant faible (voir figure 2), le processus est indépendant de l'hémolyse plasmatique.
b) Le lavage: le lavage consiste à mélanger une solution saline, introduite par le tube 21, avec les globules rouges et à évacuer le surnageant plein d'impuretés et de débris divers. Contrairement à la phase précédente de remplissage, le lavage n'est pas efficace en régime laminaire; il convient donc de profiter de la petite zone contrôlée de turbulence située à l'endroit où les deux canaux d'introduction 15 débouchent dans le bol et où les deux fluides vont se mélanger et de ramener cycliquement les globules rouges à laver dans cette zone grâce à l'effet combiné de "coups de frein" de la centrifugeuse et de la présence des deux ailettes de mélange 22. Bien entendu, ces coups de frein destabilisant momentanément la stratification des globules rouges, il faut un peu avant stopper la pompe de lavage pour éviter de perdre trop de ces globules rouges.

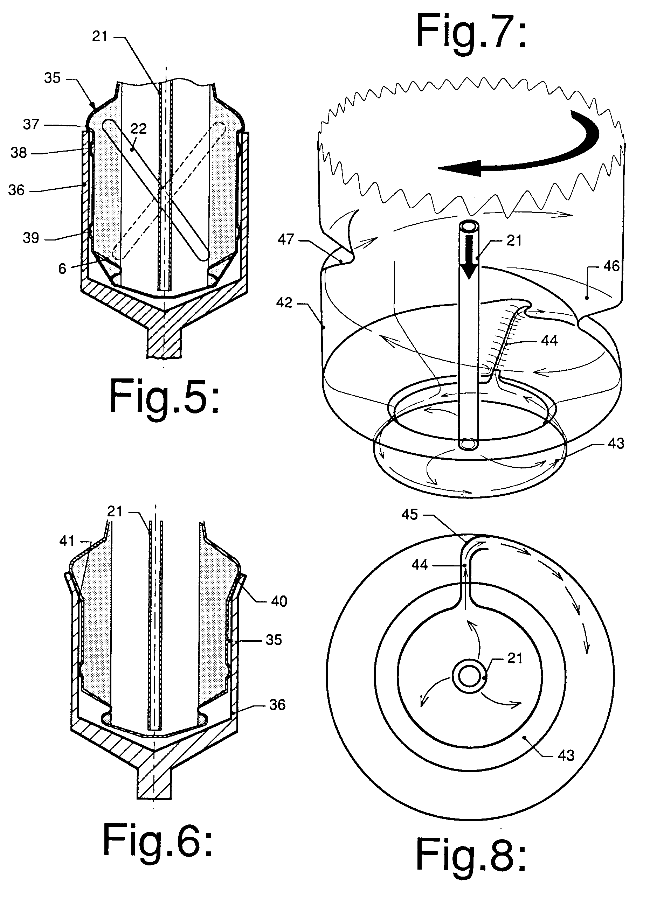

Dans la forme d'exécution du séparateur selon l'invention telle qu'illustrée partiellement sur la figure 5, la paroi principale du bol 35 présente une forme cylindrique, de même que la coupelle d'entraînement 36. Ce bol 35 présente également un épaulement supérieur périphérique 37, situé juste au-dessous de la partie conique dite inverse 8 du bol, et destiné à maintenir verticalement le bol 35 dans la coupelle 36, de telle sorte que celui-ci ne touche pas le fond de celle-ci. De plus, des tétons de centrage 38,39 sont prévus sur le pourtour des zones supérieure respectivement inférieure de la paroi cylindrique du bol 35 (environ 4 tétons répartis sur le pourtour).

D'autre part, la variante représentée sur la figure 6 comporte également un bol dont la paroi principale 35' est de forme générale cylindrique, de même que la coupelle d'entraînement 36', ainsi que des tétons élastiques de centrage inférieurs 39'. Par contre, la partie supérieure du bol de cette figure 6 se distingue de celle de la figure 5 par une portion conique de guidage 40 évasée vers le haut, servant à la fois de butée et de moyen de fixation dans la coupelle, et coopérant avec une portion supérieure 41 correspondante de ladite coupelle.

Enfin, et dans le but d'améliorer les conditions de lavage, c'est-à-dire de réaliser un mélange efficace de la solution saline et des globules rouges, sans perdre ceux-ci, une variante de la partie inférieure du bol de centrifugation a été conçue, comme représentée sur les figures 7 et 8. Avec cette variante, on évite en principe la manipulation décrite précédemment en référence à la figure 1 qui consistait à donner des "coups de frein" à la centrifugeuse. La partie inférieure 42 du bol présente un canal annulaire 43 formé par un bourrelet venu de fabrication et destiné à la stabilisation des pulsations de la pompe d'introduction. De plus, cette partie inférieure 42 comporte un canal radial unique 44 reliant le canal annulaire 43 à la partie cylindrique du bol et destiné à évacuer en l'accélérant le liquide de lavage introduit dans le canal annulaire 43 par le tube 21 sous forme d'un jet compact pénétrant les globules rouges jusqu'aux zones périphériques du fond du bol; l'écoulement étant turbulent, le liquide de lavage peut se mélanger efficacement avec les globules rouges extérieurs.

Le canal radial 44 présente en outre une portion incurvée 45 à son extrémité périphérique (dans le sens de la rotation du bol), de manière à transformer le jet radial en jet tangentiel produisant un flux en spirale montante du mélange.

Enfin, une ailette horizontale 46 est disposée au-dessus du canal radial 44, qui permet de limiter la vitesse verticale et d'éviter une sortie trop rapide risquant d'aboutir à la perte de globules rouges. Une seconde ailette 47 similaire peut être prévue au-dessus de la première, mais diamétralement opposée, et ayant le même but. Ces deux ailettes 46,47 sont de préférence également venues de fabrication avec le reste du bol.

En plus d'une évidente simplification du dispositif due notamment à l'absence totale de noyau dans le bol de centrifugation, et par là d'un prix de revient moins élevé, le séparateur centrifuge selon l'invention présente les avantages suivants par rapport aux dispositifs connus, par exemple ceux décrits dans les brevets figurant dans la partie introductive:
- la géométrie du bol de centrifugation permet d'une part de séparer les constituants du sang par exemple avec un débit élevé d'introduction grâce au régime laminaire de cette introduction, et d'autre part d'effectuer le rinçage des globules rouges de manière efficace en régime turbulent; bien que ce bol soit conçu plutôt pour des contenances relativement faibles (de l'ordre de 100 à 150 ml), le débit élevé avec lequel le traitement du fluide peut être effectué permet d'en faire un dispositif pratiquement universel, très performant, même pour des quantités importantes à traiter;
- la faible profondeur hydrostatique due au faible volume présente aussi l'avantage de limiter la pression centrifuge supportée par les globules rouges, ce qui permet ainsi de travailler avec des vitesses de rotation du bol de l'ordre de 10.000 t/min, alors que cette vitesse est généralement d'environ 6.000 t/min avec les dispositifs connus;
- la vitesse de rotation précitée est également rendue possible par une ventilation améliorée au niveau du joint rotatif et du couvercle de bol; en effet, la rotation de l'ensemble coupelle - bol - couvercle de bol crée un appel d'air par le haut (orifices 18) et une sortie d'air par le bas (passages 25) du séparateur, de manière à former un courant destiné à refroidir ledit ensemble; de plus, le couvercle de bol réalisé en acier inox à haute conductivité thermique conduit la chaleur vers l'extérieur où elle est évacuée par la convection due aux fortes vitesses périphériques;
- le joint rotatif souple en élastomère frottant contre le couvercle de bol assure l'étanchéité d'une manière efficace et beaucoup moins onéreuse que les joints utilisés dans les dispositifs connus, qui sont généralement constitués par deux pièces rigides de plastique graphité qui doivent être parfaitement rectifiées et planées;
- enfin, la conception décrite ci-dessus de contrôle du passage des premiers globules rouges par fibre optique, au lieu de la détection de la transition de couleur entre les globules rouges et le plasma qui est utilisée dans les dispositifs connus, permet d'obtenir des hématocrites de valeur élevée, de l'ordre de 80%, contre 40-60% pour ces dispositifs connus; ceci a en outre l'avantage de réduire le volume de liquide de lavage nécessaire, et ainsi de réduire la consommation d'eau de lavage.

## Revendications

1. Séparateur centrifuge pour fluides comportant une enceinte dans laquelle est montée rotative une coupelle d'entraînement (3), un bol de centrifugation (5) jetable en un matériau plastique, présentant une forme externe correspondant à celle de la coupelle et introduit à force en position de service dans ladite coupelle d'entraînement, un couvercle (16) coopérant avec l'enceinte pour fermer celle-ci au-dessus dudit bol, un tube d'introduction (21) du fluide à traiter disposé verticalement dans ce bol, de telle sorte que son extrémité ouverte débouche a proximité du fond (7) dudit bol de centrifugation, ainsi que deux éléments circulaires coniques (28,28') formant des lèvres d'aspiration, l'espace entre celles-ci communiquant avec un conduit d'évacuation (19), caractérisé par le fait que ledit bol de centrifugation (5) présente deux bourrelets annulaires, respectivement supérieur (14) et inférieur (6), dans chacun desquels sont pratiqués des passages verticaux (14',15), par le fait que ce bol est fermé à son extrémité périphérique supérieure par un couvercle (11) métallique à travers lequel le tube d'introduction (21) est disposé solidairement, et par le fait qu'un joint rotatif souple (17) solidaire du couvercle (16) du séparateur est en contact avec ledit couvercle de bol (11).

2. Séparateur selon la revendication 1, caractérisé par le fait que la coupelle d'entraînement (3) et le bol de centrifugation (5) sont de forme générale conique s'évasant vers le haut.

3. Séparateur selon la revendication 1, caractérisé par le fait que la coupelle d'entraînement (36) et le bol de centrifugation (35) sont de forme générale cylindrique, et par le fait que le bol présente sur sa paroi cylindrique externe des formations de positionnement vertical (37) et de centrage (38,39) coopérant en position de service avec la paroi cylindrique interne de ladite coupelle.

4. Séparateur selon l'une des revendications 1 à 3, caractérisé par le fait que l'enceinte est formée de deux parties superposées (1a,1b) et reliées l'une à l'autre par des moyens élastiques (2) servant de suspension anti-vibratoire.

5. Séparateur selon l'une des revendications 1 à 4, caractérisé par le fait que le bol de centrifugation (5) présente un fond (7) légèrement conique, une partie principale conique évasée vers le haut ou cylindrique, deux portions coniques (9) adjacentes se rétrécissant vers le haut et une seconde portion conique (10) évasée vers le haut, le bourrelet supérieur (14) étant situé entre la seconde portion conique se rétrécissant (9) et la seconde portion conique évasée (10).

6. Séparateur selon la revendication 5, caractérisé par le fait que le couvercle de bol (11) est fixé par sa périphérie à l'extrémité ouverte de ladite seconde portion conique (10) au moyen d'une bague de fixation intermédiaire (12).

7. Séparateur selon l'une des revendications 1 à 6, caractérisé par le fait que des ailettes de mélange (22) sont venues de fabrication à l'intérieur du bol de centrifugation (5).

8. Séparateur selon l'une des revendications 1 à 6, caractérisé par le fait que la partie inférieure (42) du bol comporte un canal annulaire (43) et un canal radial (44) reliant le canal annulaire avec la partie supérieure du bol, la portion d'extrémité périphérique de ce canal radial étant incurvée dans le sens de rotation du bol.

9. Séparateur selon la revendication 8, caractérisé par le fait qu'au moins une ailette horizontale (46) est disposée au-dessus du canal radial (44), et par le fait que le canal annulaire, le canal radial de liaison et la ou les ailette(s) horizontale(s) sont venus de fabrication avec le bol.

10. Séparateur selon l'une des revendications 1 à 9, caractérisé par le fait que le couvercle du séparateur (16) comporte des moyens de fixation du type à baïonnette destinés à coopérer avec des moyens correspondants que présente la partie supérieure de l'enceinte du séparateur, et par le fait que des ouvertures de ventilation (12) sont pratiquées dans ce couvercle.

11. Séparateur selon l'une des revendications 1 à 10, caractérisé par le fait qu'il comporte un dispositif de contrôle comprenant un faisceau optique formé horizontalement entre un émetteur (26) et un récepteur (27), juste au-dessous dudit bourrelet supérieur (9) du bol (5) et de manière légèrement décentrée axialement.

12. Séparateur selon l'une des revendications 1 à 11, caractérisé par le fait que lesdits éléments circulaires coniques (28,28') formant les lèvres d'aspiration présentent des angles de conicité différents, de telle sorte que l'espace entre ces deux éléments diminue en direction de leur périphérie.

## Claims

1. A centrifugal separator for fluids comprising a housing inside which is mounted a rotatable driving cup, a disposable centrifugal bowl made of a plastic material which has an external shape corresponding to that of the cup and which is press fitted before operation inside said driving cup, a cover cooperating with the housing to close the same above said bowl, a supplying tube of the fluid to be treated being positioned vertically inside said bowl, so that its open end be located close to the bottom of said centrifugal bowl, as well as two conical circular elements forming suction lips, the space between them communicating with a discharge conduit, characterized in that said centrifugal bowl has two annular beads, respectively an upper one and a lower one, each one being provided with vertical passages, in that this bowl is closed at its peripheral upper end by a metallic bowl cover through which the supplying tube is jointly arranged, and in that a flexible rotatable seal integral with the cover of the separator is in contact with said bowl cover.

2. A separator according to claim 1, characterized in that the driving cup and the centrifugal bowl have a generally conical shape widening upwardly.

3. A separator according to claim 1, characterized in that the driving cup and the centrifugal bowl are of a generally cylindrical shape, and in that the bowl has on its outer cylindrical wall structures for ensuring its vertical positioning and centering and for cooperating in the operative position with the internal cylindrical wall of said cup.

4. A separator according to one of claims 1 to 3, characterized in that the housing is formed of two superposed parts connected together by resilient means acting as a vibration damper.

5. A separator according to one of claims 1 to 4, characterized in that the centrifugal bowl has a bottom which is slightly conical, a main portion which is either conical and widens upwardly, or cylindrical, two adjoining conical portions narrowing upwardly and a second conical portion widening upwardly, with the upper bead being located between the second conical narrowing portion and the second widening conical portion.

6. A separator according to claim 5, characterized in that the cover of the bowl is fastened at its periphery to the open end of said second conical portion by means of an intermediate ring fastener.

7. A separator according to one of claims 1 to 6, characterized in that mixing blades integral with the bowl are provided inside the centrifugal bowl.

8. A separator according to one of claims 1 to 6, characterized in that the lower part of the bowl is provided with an annular channel and with a radial channel connecting the annular channel to the upper part of the bowl, the peripheral end portion of this radial channel being curved in the direction of rotation of the bowl.

9. A separator according to claim 8, characterized in that at least one horizontal blade is positioned above the radial channel and in that the annular channel, the radial connecting channel and the horizontal blade or blades are integral with the bowl.

10. A separator according to one of claims 1 to 9, characterized in that the cover of the separator includes fastening means of the bayonet type designed for cooperating with corresponding means provided on the upper part of the housing of the separator and in that ventilation openings are provided in this cover.

11. A separator according to one of claims 1 to 10, characterized in that it includes a control device with a light beam travelling horizontally between an emitter and a receiver, immediately beneath said upper bead of the bowl and slightly offset with respect to the bowl axis.

12. A separator according to one of claims 1 to 11, characterized in that said circular conical components acting as suction lips have different angles of taper, so that the distance between these two components decreases in the direction of their periphery.

## Patentansprüche

1. Zentrifugalseparator für Fluide mit einem Behältnis, in das drehbar eine Antriebstrommel (3), ein Einweg-Zentrifugiermantel (5) aus Kunststoff, der eine der Antriebstrommel entsprechende äussere Gestalt aufweist und unter Kraftanwendung in die Arbeitsstellung in der benannten Trommel eingepasst wird, einen Deckel (16), der mit dem Behältnis zusammenwirkt, um dieses oberhalb des benannten Mantels abzuschliessen, ein Einführungsrohr (21) für das zu behandelnde Fluid, das senkrecht so in diesem Mantel angeordnet ist, dass sein offenes Ende in der Nähe des Bodens (7) des benannten Zentrifugiermantels mündet, sowie zwei kreisrunde, konische Elemente (28, 28') eingebaut sind, die Ansauglippen bilden, wobei der Baum zwischen ihnen mit einem Entleerungsrohr (19) in Verbindung steht, dadurch gekennzeichnet, dass der benannte Zentrifugiermantel (5) zwei ringförmige Wülste, einen oberen (14) und einen unteren (6), aufweist, wobei in jedem dieser Wülste senkrechte Durchlässe (14', 15) angebracht sind, dadurch, dass dieser Mantel an seinem oberen peripheren Ende von einem Metalldeckel (11) verschlossen wird, durch den das fest mit ihm verbundene Einführungsrohr (21) führt, und dadurch, dass eine weiche Drehdichtung (17), die mit dem Deckel (16) des Separators fest verbunden ist, mit dem benannten Deckel (11) des Mantels in Berührung steht.

2. Separator gemäss Anspruch 1, dadurch gekennzeichnet, dass die Antriebstrommel (3) und der Zentrifugiermantel (5) von allgemein konischer Form sind und sich nach oben hin erweitern.

3. Separator gemäss Anspruch 1, dadurch gekennzeichnet, dass die Antriebstrommel (36) und der Zentrifugiermantel (35) von allgemein zylindrischer Form sind und dass der Mantel an seiner zylindrischen Aussenwand Elemente zur senkrechten Positionierung (37) und Zentrierung (38, 39) aufweist, die in der Arbeitsstellung mit der zylindrisches Innenwand der benannten Trommel zusammenwirken.

4. Separator gemäss einem der Ansprüche 1 bis 3, dadurch gekennzeichnet, dass das Behältnis von zwei übereinandergesetzten Teilen (1*a*, 1*b*) gebildet wird, die miteinander durch elastische Organe (2) verbunden sind, die als erschütterungsdämpfende Aufhängung dienen.

5. Separator gemäss einem der Ansprüche 1 bis 4, dadurch gekennzeichnet, dass der Zentrifugiermantel (5) einen leicht konischen Boden (7), einen konischen, nach oben hin ausgeweiteten oder zylindrischen Hauptteil, zwei aneinandergrenzende, sich nach oben hin verjüngende konische Abschnitte (9) sowie einen zweiten, nach oben hin ausgeweiteten konischen Abschnitt (10) aufweist, wobei sich der obere Wulst (14) zwischen dem zweiten, sich verjüngenden konischen Abschnitt (9) und dem zweiten, ausgeweiteten konischen Abschnitt (10) befindet.

6. Separator gemäss Anspruch 5, dadurch gekennzeichnet, dass der Manteldeckel (11) an seinem Umfang über einen Befestigungszwischenring (12) am offenen Ende des benannten zweiten konischen Abschnitts (10) befestigt ist.

7. Separator gemäss einem der Ansprüche 1 bis 6, dadurch gekennzeichnet, dass Rührflügel (22) an der Innenseite des Zentrifugiermantels (5) zusammen mit diesem gefertigt worden sind.

8. Separator gemäss einem der Ansprüche 1 bis 6, dadurch gekennzeichnet, dass der untere Teil (42) des Mantels einen Ringkanal (43) sowie einen Radialkanal (44) umfasst, der den Ringkanal mit dem oberen Teil des Mantels verbindet, wobei der periphere Endabschnitt dieses Radialkanals im Drehsinn des Mantels abgekrümmt ist.

9. Separator gemäss Anspruch 8, dadurch gekennzeichnet, dass zumindest ein waagerechter Flügel (46) oberhalb des Radialkanals (44) angeordnet ist, und dass der Ringkanal, der radiale Verbindungskanal und der (die) waagerechte(n) Flügel mit dem Mantel zusammen gefertigt worden sind.

10. Separator gemäss einem der Ansprüche 1 bis 9, dadurch gekennzeichnet, dass der Deckel des Separators (16) Befestigungsorgane von Bajonetttyp umfasst, die dazu bestimmt sind, mit entsprechenden Organen zusammenzuwirken, die der obere Teil des Separatorbehältnisses aufweist, und dadurch, dass Lüftungsöffnungen (12) in diesem Deckel angebracht sind.

11. Separator gemäss einem der Ansprüche 1 bis 10, dadurch gekennzeichnet, dass er eine Steuervorrichtung mit einem optischen Strahlenbündel aufweist, das waagerecht zwischen einem Sender (26) und einem Empfänger (27) gerade unterhalb des benannten oberen Wulstes (9) des Mantels und axial geringfügig exzentrisch ausgebildet ist.

12. Separator gemäss einem der Ansprüche 1 bis 11, dadurch gekennzeichnet, dass die benannten kreisrunden, konischen Elemente (28, 28'), die die Ansauglippen bilden, verschiedene Kegelsteigungen aufweisen, so dass sich der Baum zwischen diesen beiden Elementen in Richtung auf ihre Peripherie verjüngt.
